# EUROPÄISCHE PATENTANMELDUNG

(11) **EP 2 039 351 A1**
(43) Veröffentlichungstag der Anmeldung: **25.03.2009**
(21) Anmeldenummer: 08016664.8
(22) Anmeldetag: 23.09.2008
(51) Int. Cl.: A61K 9/16

(54) **Verfahren zur Herstellung frei fließender, pulverförmiger Extrakte mit Hilfe der Wirbelschicht Trocknung**

(30) Priorität: 24.09.2007 DE 102007045686
(71) Anmelder: J. Rettenmaier & Söhne GmbH + Co. KG, 73494 Rosenberg (DE)
(72) Erfinder: Stoyanov, Edmont, Dr., 73433 Aalen (DE); Götz, Tobias, 73494 Rosenberg (DE); Vollmer, Reinhard, Dr., 55452 Windesheim (DE)
(74) Vertreter: Weitzel, Wolfgang

(57) **Zusammenfassung**

Die Erfindung beinhaltet einen frei fließenden Extrakt, aus pflanzlichem, tierischem oder anderem biologischen Material, **dadurch gekennzeichnet, dass** der Extrakt vor oder nach der Trockung mit Natriumstearylfumarat überzogen und anschließend auf eine Mischung aus silifizierter Mikrokristalliner Cellulose und Natriumcarboxymethyl-stärke oder ein enderes Sprengmittel (z. B. Croscarmellose Natrium, PVPP etc.) aufgesprüht wird.

## Beschreibung

Getrocknete Extrakte, aus Pflanzen, Tieren oder anderem biologischen Material sind meistens klebrig, nicht frei fließend und somit nur schwer zu Tabletten verpressbar. Um mit solchen Extrakten in direkter Verpressung Tabletten herzustellen, ist die Zugabe von Bindemitteln, Zerfallsbeschleunigern und Schmiermitteln notwendig.

Es hat Versuche gegeben, im Sprühtrockner halbflüssige Spissum Extrakte auf silicifizierte Mikrokristalline Cellulose aufzusprühen. Dabei entstehen allerdings noch keine direkt verpressbaren Pulver, der Zusatz von Spreng- und Schmiermitteln ist bei dieser Art Extrakten weiterhin notwendig. Eine solche Anwendung findet sich in US 2003/0203050A1, Oct.30, 2003

In der Pharmazeutischen Industrie besteht nach wie vor Bedarf an frei fließenden Extrakten, die ohne Zugabe weiterer Hilfsstoffe direkt zu Tabletten verpresst werden können. Dabei werden möglichst kleine Tablettengrößen, ausreichenden Härten und kurze Zerfallszeiten gefordert.
1. Ausgangsmaterial ist ein Extrakt in Spissum oder Trocken Form sowie ein Fruchtsaftkonzentrat.
2. Der Extrakt wird in der Folge auf den Trägerstoff, mikrokristalline Cellulose oder silicifizierte Mikrokristalline Cellulose, aufgetragen. Die gleichmäßige Verteilung des Extraktes sowie die Binde- und Fließfähigkeit werden erhöht, wenn der Extrakt mit Natriumstearylfumarat überzogen ist.
3. Dazu wird Natriumstearylfumarat in heißem Wasser gelöst und sorgfältig gerührt. In diese Lösung lässt man den Extrakt einfließen und setzt das Rühren fort, bis optisch eine homogene Lösung oder Suspension entstanden ist.
4. Parallel werden in einem Wirbelschichttrockner silicifizierte Mikrokristalline Cellulose und Natriumcarboxymethylstärke oder ein anderes Sprengmittel (z. B. Croscarmellose Natrium, PVPP etc.) trocken vorgelegt und auf ca. 40 °C aufgeheizt. Dabei ist auf einen kräftigen Luftstrom von unten zu achten.
5. Mittels einer obenliegenden Düse sprüht man in der Wirbelschicht den Extrakt aus 3. langsam auf die silicifizierte Mikrokristalline Cellulose oder mikrokristalline Cellulose aus 4. auf. Die Sprührate muss so dosiert werden, dass sich keine Klumpen bilden.
6. Durch heiße Luft von mindestens 60 °C wird das Pulvergemisch getrocknet. Der trockene Extrakt wird dem Wirbelschichttrockner entnommen.

Beispiel einer Extraktherstellung, hier Baldrian Extrakt
A. 1,0 g Natriumstearylfumarat (PRUV^{®}) werden in 200 ml Wasser, demin. bei ca. 90 °C restlos gelöst.
B. In die noch heiße Lösung von Natriumstearylfumarat werden 48,0 g Baldrian Trockenextrakt eingegeben und gelöst.
C. Im Wirbelschichttrockner mit obenliegender Düse werden 48,0 g silizifizierte Mikrokristalline Cellulose und 3,0 g Natriumcarboxymethylstärke vorgelegt und aufgeheizt.
D. Die Lösung von Natriumstearylfumarat und Baldrian Extrakt wird auf die silizifizierte Mikrokristalline Cellulose und die Natriumcarboxymethylstärke aufgesprüht.
E. Mit heißer Luft wird die Pulvermischung auf einen Feuchtegehalt von 4,4-4,6 % getrocknet.
F. Die Pulvermischung wird direkt zu Tabletten verpresst.

Die Formulierung:

| | | |
|---|---|---|
| Baldrian Extrakt | 48.00 % | |
| PROSOLV SMCC^{®} 90 | 48.00 % | (silizifizierte Mikrokristalline Cellulose) |
| EXPLOTAB^{®} CLV | 3.00 % | (Natriumcarboxymethylstärke) |
| PRUV^{®} | 1.00 % | (Natriumstearylfumarat) |

### Pressdaten:

| Tablettenpresse | Kilian Pressima |
|---|---|
| Presskraft | 9 kN |
| Tablettenhöhe | 3.6 mm |
| Tablettendurchmesser | 13 mm |
| Tablettenhärte | 7-8 kp |
| Tablettengewicht | 600 mg +/-4 mg |
| Abrieb | 0.17 % |
| Zerfallszeit | 11 min |

### Wirbelschichtdaten

| | |
|---|---|
| Gebläse ca. | 60 % gegen später erhöht (low Air Flow) |
| Lufttemperatur | 70-80 °C |
| Produkttemperatur | - 40 °C |
| Sprühlanzendruck | 0.5 Bar |
| Pumpgeschwindigkeit | 20 U/min |

## Patentansprüche

1. Verfahren zur Herstellung eines trockenen, frei fließenden Extraktes aus pflanzlichem oder tierischem Material sowie aus Fruchtsaftkonzentraten, **dadurch gekennzeichnet, dass** der noch flüssige oder schon trockene Extrakt in einer Lösung aus Natriumstearylfumarat in Wasser gelöst und anschließend auf microkristalline Cellulose oder silizifizierte mikrokristalline Cellulose und Natriumcarboxymethylstärke oder ein enderes Sprengmittel (z. B. Croscarmellose Natrium, PVPP etc.) aufgesprüht wird.

2. Ein direkt verpressbarer Extrakt, **dadurch gekennzeichnet, dass** zur Verpressung desselben keine weiteren Hilfsstoffe, wie z.B. Bindemittel oder Schmiermittel zugesetzt werden müssen.
